# EUROPÄISCHE PATENTSCHRIFT

(11) **EP 1 167 541 B1**
(45) Veröffentlichungstag und Bekanntmachung des Hinweises auf die Patenterteilung: **10.10.2007**
(21) Anmeldenummer: 01114412.8
(22) Anmeldetag: 15.06.2001
(51) Int. Cl.: C12Q 1/68

(54) **Verfahren zur Ermittlung von Daten zur präsymptomatischen oder pränatalen Diagnose von Typ 1 Neurofibromatose**
Method for obtaining data for the presymptomatic or prenatal diagnosis of neurofibromatosis typ 1
Procédé de d'obtention de données pour le diagnostique pré-symptomatique ou pré-natal de la neurofibromatose de type 1

(30) Priorität: 27.06.2000 EP 00113607
(43) Veröffentlichungstag der Anmeldung: 02.01.2002
(73) Patentinhaber: Von Recklinghausen Gesellschaft E.V., 20246 Hamburg (DE)
(72) Erfinder: Kluwe, Lan, Dr.rer. nat, 21035 Hamburg (DE)
(74) Vertreter: Eisenführ, Speiser & Partner

(56) Entgegenhaltungen:
- WU C L ET AL: "Differential diagnosis of type 2 neurofibromatosis: molecular discrimination of NF2 and sporadic vestibular schwannomas." JOURNAL OF MEDICAL GENETICS. ENGLAND DEC 1998, Bd. 35, Nr. 12, Dezember 1998 (1998-12), Seiten 973-977, XP008019307 ISSN: 0022-2593
- KLUWE LAN ET AL: "Allelic loss of the NF1 gene in NF1-associated plexiform neurofibromas." CANCER GENETICS AND CYTOGENETICS, Bd. 113, Nr. 1, August 1999 (1999-08), Seiten 65-69, XP000961009 ISSN: 0165-4608
- KLUWE ET AL.: "Loss of NF1 allele in Schwann cells but not in fibroblasts derived from NF1-associated neurofibroma" GENES,CHROMOSOMES & CANCER, Bd. 24, 1999, Seiten 283-285, XP000961010
- RIVA ET AL.: "Characterization of cytogenetic 17q11.2 deletion in an NF1 patient with a contiguous gene syndrome" HUM.GENET, Bd. 98, 1996, Seiten 646-650, XP000961008
- CLEMENTI M ET AL: "Clinical application of genetic polymorphism in neurofibromatosis type 1." ANNALES DE GENETIQUE. FRANCE 1996, Bd. 39, Nr. 2, 1996, Seiten 92-96, XP008019271 ISSN: 0003-3995
- BASER M E ET AL: "Presymptomatic diagnosis of neurofibromatosis 2 using linked genetic markers, neuroimaging, and ocular examinations." NEUROLOGY, Bd. 47, Nr. 5, 1996, Seiten 1269-1277, XP000961288 ISSN: 0028-3878
- SAINIO M ET AL: "Presymptomatic DNA and MRI diagnosis of neurofibromatosis 2 with mild clinical course in an extended pedigree." NEUROLOGY, Bd. 45, Nr. 7, 1995, Seiten 1314-1322, XP000961293 ISSN: 0028-3878
- KLUWE L ET AL: "The parental origin of new mutations in neurofibromatosis 2." NEUROGENETICS. ENGLAND SEP 2000, Bd. 3, Nr. 1, September 2000 (2000-09), Seiten 17-24, XP002249740 ISSN: 1364-6745
- SAINZ JESUS ET AL: "Loss of alleles in vestibular Schwannomas: Use of microsatellite markers on chromosome 22." ARCHIVES OF OTOLARYNGOLOGY HEAD & NECK SURGERY, Bd. 119, Nr. 12, 1993, Seiten 1285-1288, XP000961281 ISSN: 0886-4470
- ROHDE K ET AL: "Analysis of genetic linkage and somatic loss of heterozygosity in affected pairs of first-degree relatives." AMERICAN JOURNAL OF HUMAN GENETICS. UNITED STATES AUG 1997, Bd. 61, Nr. 2, August 1997 (1997-08), Seiten 418-422, XP008019287 ISSN: 0002-9297
- LASKO D ET AL: "LOSS OF CONSTITUTIONAL HETEROZYGOSITY IN HUMAN CANCER" ANNUAL REVIEW OF GENETICS, 1992, Seiten 281-314, XP008019292 ISSN: 0066-4197
- DATABASE BIOSIS [Online] BIOSCIENCES INFORMATION SERVICE, PHILADELPHIA, PA, US; FORTSCHRITTE DER NEUROLOGIE PSYCHIATRIE, Juni 1998 (1998-06) MAUTNER V -F ET AL: "Neurofibromatosis versus schwannomatosis." XP002154234
- KLUWE LAN ET AL: "Presymptomatic diagnosis for children of sporadic neurofibromatosis 2 patients: a method based on tumor analysis." GENETICS IN MEDICINE: OFFICIAL JOURNAL OF THE AMERICAN COLLEGE OF MEDICAL GENETICS. UNITED STATES 2002 JAN-FEB, Bd. 4, Nr. 1, Januar 2002 (2002-01), Seiten 27-30, XP008020246 ISSN: 1098-3600

## Beschreibung

Die Erfindung betrifft ein Verfahren zur Ermittlung von Daten zur Vorbereitung der präsymptomatischen oder pränatalen Diagnose von Typ 1 Neurofibromatosen.

Derartige Verfahren sind aus dem Stand der Technik bekannt. Sie dienen vor allem dazu, bei Kindern von an einer Erbkrankheit erkrankten Eltern oder deren Enkelkindern die Wahrscheinlichkeit des erneuten Auftretens der Erbkrankheit frühzeitig, u.a. auch (möglichst) pränatal beurteilen zu können. Im Stand der Technik wurden zur Vorbereitung der entsprechenden Diagnose zu diesem Zweck eine Mutationsanalyse des entsprechenden, für das die Erbkrankheit charakterisierende Gen kodierenden DNA-Abschnittes durchgeführt. Eine dieser Tumorsuppressorgenerkrankungen sind die autosomal dominant vererbten Neurofibromatosen. Die Neurofibromatose tritt in zwei Typen auf, dem "peripheren Typ", Typ 1 genannt, der etwa 85% der Fälle ausmacht und dem "zentralen Typ", Typ 2 genannt, der etwa 15% der Fälle ausmacht. Der Typ 1 tritt mit einer Inzidenz von etwa 1:3000 auf, während der Typ 2 mit einer Inzidenz von etwa 1:35000 auftritt. Bezüglich des klinischen Erscheinungsbildes sei auf die einschlägigen medizinischen Fachbücher verwiesen.

Nachteilig bei der aus dem Stand der Technik bekannten Mutationsanalyse ist es jedoch, daß diese sehr zeitaufwendig ist. So weist beispielsweise das Neurofibromatose Typ 1-Gen auf dem Chromosom 17 (NF1-Gen) 60 Exons auf. Eine komplette Untersuchung dieses Gens dauert mit Hilfe der bekannten Mutationsanalyse mehr als vier Monate. Das auf dem Chromosomen 22 liegende Neurofibromatose Typ 2-Gen (NF2-Gen) ist zwar kleiner, da es nur 17 Exons aufweist; eine komplette Untersuchung dauert hier jedoch ebenfalls länger als einen Monat. Darüber hinaus ist es bei der bekannten Mutationsanalyse nachteilig, daß bei den Risikopersonen erst dann eine molekulargenetisch abgesicherte Diagnose möglich ist, wenn eine Mutation bei den Betroffenen gefunden wurde.

Ein Verfahren der eingangs genannten Art ist beispielsweise auch aus dem Aufsatz von C.L.Wu et al. "Differential Diagnosis of Type 2 Neurofibromatosis: Molecular Discrimination of NF2 and sporadic vestibular schwannomas" Journal of Medical Genetics, Band 35, Nr. 12, Dezember 1998 (1998-12) Seiten 973-977, XP008019307 ISSN: 0022-2593 bekannt. Bei diesem bekannten Verfahren wird bei Patienten, bei denen nicht eindeutig feststeht, ob sie unter Neurofibromatose leiden, Tumormaterial von mindestens zwei Tumoren dieser Patienten und Blut dieser Patienten untersucht, um festzustellen, ob diese Patienten an Neurofibromatose erkrankt sind. Hierzu wird nach einer entsprechenden Mutation in der DNA des Patienten gesucht. Weiterhin offenbart dieser Stand der Technik, dass dann, wenn eine Erkrankung bei dem Patienten festgestellt wurde, bei Kindern des Patienten das Risiko einer Erkrankung ausgeschlossen werden kann, wenn diese Kinder nicht die gleichen Mutationen zeigen. Wie bereits oben dargelegt, sind derartige Mutationsanalysen jedoch nachteilig, da sie nur dann eine vernünftige Risikoeinschätzung ermöglichen, wenn eine Mutation bei den Kindern aufgefunden wird. Wird jedoch keine Mutation aufgefunden, ist keine gesicherte Risikoabschätzung für Kinder von dem Patienten möglich.

Aus dem Aufsatz "CLINICAL APPLICATION OF GENETIC POLYMORPHISM IN NEUROFIBROMATOSIS TYPE 1" von M. Clementi, Stefania Boni, Isabella Mammi, M. Favarato und R. Tenconi aus der Zeitschrift Annales de Génétique, Frankreich, 1996, Band 39, Nr. 2, Seiten 92-96, XP008019271, ISSN: 0003-3995 ist ein Verfahren bekannt, bei dem als krank erkannte Individuen aus NF1 Familien untersucht worden sind. Dabei wurde DNS aus Blut isoliert und per PCR auf eine Reihe von Polymorphismen untersucht.

Aufgabe der vorliegenden Erfindung ist es daher, ein Verfahren der eingangs genannten Art zu verbessern.

Diese Aufgabe wird bei einem Verfahren der eingangs genannten Art erfindungsgemäß durch ein Verfahren gemäß Anspruch 1 gelöst.

Die Vorteile des erfindungsgemäßen Verfahrens liegen insbesondere darin, daß es sehr schnell durchgeführt werden kann. So kann das erfindungsgemäße Verfahren auf jeden Fall in längstens zwei Wochen durchgeführt werden; es kann jedoch bei beschleunigter Durchführung auch in etwa zwei Tagen durchgeführt werden. Diese Schnelligkeit des erfindungsgemäßen Verfahrens ist besonders in der pränatalen Diagnostik wichtig. Darüber hinaus ist das erfindungsgemäße Verfahren aufgrund seiner Einfachheit auch erheblich kostengünstiger als die aus dem Stand der Technik bekannte Mutationsanalyse.

Als besonders vorteilhaft bei dem erfindungsgemäßen Verfahren hat es sich erwiesen, daß in den Fällen, bei denen bei der bekannten Mutationsanalyse keine Mutation bei den Betroffenen gefunden werden konnte, das erfindungsgemäße Verfahren nunmehr bei sporadischen Fällen die einzige Möglichkeit bietet, Neurofibromatosen vom Typ 1 auf molekularer Ebene auszuschließen oder zu bestätigen. Dabei ist insbesondere der Ausschluß der Neurofibromatose von besonderer Bedeutung, denn statistisch gesehen kann bei etwa 50% der Risikopersonen die Krankheit ausgeschlossen werden. Dadurch können aufgrund der Erfindung nicht nur kostenintensive Mutationsanalysen und Untersuchungen eingespart werden, sondern es wird den untersuchten Risikopersonen auch die Angst genommen, die Krankheit ererbt zu haben. Auch werden kostspielige klinische Untersuchungen vermieden.

Bei einer bevorzugten Ausführungsform handelt es sich bei den Markern um relativ kurze genflankierende oder intragenische DNA-Abschnitte (bis 300 bp). Dies bietet den Vorteil, daß beispielsweise in Form eines Paraffinblockes vorliegendes Tumormaterial, welches beispielsweise aufgrund einer Operation von Hauttumoren bei Neurofibromatose gewonnen wurde, verwendet werden kann, da es möglich ist, kurze DNA-Abschnitte aus den meisten üblicherweise vorhandenen Paraffinblöcken zu vermehren. Als besonderer Vorteil ist es dabei anzusehen, daß insbesondere bei Neurofibromatosen das Tumormaterial leicht durch äußere Eingriffe gewonnen werden kann.

Bei einer weiteren bevorzugten Ausführungsform werden mindestens vier verschiedene Marker vermehrt. Auf diese Weise wird für die spätere Diagnose eine bessere und mögliche Fehlbeurteilungen nachteilig ausschließende Datenbasis geschaffen.

Bei einer weiteren vorteilhaften Ausführungsform der Erfindung wird die Diagnose der Neurofibromatose vom Typ 1 vorbereitet. Zu diesem Zweck wird mindestens ein polymorpher Mikrosatelliten-Marker aus dem Intron 27 des NF1-Gens verwendet. Weiterbevorzugt wird mindestens ein weitererpolymorpher Mikrosatelliten-Marker aus dem Intron 38 des NF1-Gens verwendet. Optimale Ergebnisse lassen sich erzielen, wenn insgesamt drei oder vier Marker aus den genannten Introns verwendet werden. Dies ist vorteilhaft, da es sich gezeigt hat, daß bei der überwiegenden Vielzahl der untersuchten Risikopatienten mindestens einer der genannten Marker informativ ist. Informativ ist ein Marker bei einer Person, wenn der entsprechende DNA-Abschnitt polymorph und in zwei unterschiedlichen Längen auf den beiden Kopien des Erbgutes vorliegt. Durch die genannten Marker wird somit mit hoher Wahrscheinlichkeit sichergestellt, daß sich in der graphischen Darstellung der Marker aufgrund der unterschiedlichen Länge zwei Spitzen ergeben.

Als vorbereitender Schritt für die Diagnose kann dann ein Arzt die beiden Spitzen der graphischen Darstellung der Marker aus dem Blut des Betroffenen zunächst mit dem Ergebnis der graphischen Auftragung der Länge der DNA-Mikrosatelliten-Marker aus dem Tumor vergleichen, um einen möglichen LOH (loss of heterocygosity = LOH) festzustellen. Dabei schließt die Erfindung die Erkenntnis ein, daß die Neurofibrome der Personen, denen das Tumormaterial entnommen wurde, zu 30% einen Verlust der Heterozygotie aufweisen, wenn es sich um Neurofibromatose Typ 1 handelt. Bei Neurofibromatose Typ 2-assoziierten Tumoren ist die LOH-Rate noch höher angesiedelt. Somit und aufgrund der Tatsache, daß NF1-Patienten viele Neurofibrome aufweisen, ist die Wahrscheinlichkeit sehr hoch, daß LOH in irgendeinem der Neurofibrome des Erkrankten vorliegt, und somit auch in dem zur Verfügung gestellten Tumormaterial vorliegt und erkannt werden kann. Der LOH ist dann in der graphischen Darstellung der Marker daran erkennbar, daß bei dem Tumormaterial nur eine Spitze oder ein Ungleichgewicht der beiden Spitzen des entsprechenden Markers zu erkennen ist. Beides bedeutet, daß der entsprechende Tumor ein Allel verloren hat. Nach der Feststellung eines LOH wird dann der gleiche Marker aus dem Blut der Risikoperson untersucht.

Bei einer anderen vorteilhaften Ausführungsform der Erfindung werden die Schritte c), e), g), i) mindestens einmal wiederholt. Auf diese Weise kann ein Allelverlust erneut überprüft bzw. bestätigt werden. Es kann somit eine Absicherung des ersten Ergebnisses erzielt werden, wenn bei einem LOH das verlorene Allel auch in der mindestens einen weiteren Untersuchung übereinstimmt.

Bei einer weiteren bevorzugten Ausführungsform wird ein solcher LOH möglichst an mindestens einem weiteren Tumor des Betroffenen überprüft, d.h. es werden die vorgenannten Schritte mit mindestens einem weiteren Tumor des Betroffenen durchgeführt, wenn dieser zur Verfügung steht. Auf diese Weise läßt sich die Zuverlässigkeit der erhaltenen Daten weiter erhöhen. Dies ist insbesondere in der pränatalen Diagnostik von Vorteil.

Ein weiteres Ausführungsbeispiel der Erfindung führt die Schritte b), d), f), h) und j) auch mit dem Blut des nicht betroffenen Elternteils durch, wenn es sich bei dem Risikopatienten um ein Kind beider Elternteile handelt. Auf diese Weise lassen sich nicht betroffene Allele ermitteln. Dies dient einerseits ebenfalls der Erhöhung der Zuverlässigkeit der erhaltenen Daten und ist andererseits in gewissen Fällen bei der Auswertung der erhaltenen Daten für die Diagnose unabdingbar. Als Beispiel sei diesbezüglich erwähnt, dass es möglich ist, dass sich bei der graphischen Darstellung der Allele des Betroffenen zeigt, dass dieser die Allele A und B besitzt. Bei der graphischen Darstellung der Allele des Risikopatienten, d.h. in diesem Fall des Kindes des Betroffenen zeigt sich, dass auch das Kind die Allele A und B besitzt. Bei der graphischen Darstellung des Tumormaterials des Betroffenen zeigt sich, dass im Tumor das Allel A verlorengegangen ist. In einem solchen Falle wäre die gewonnenen Daten somit eine unklare Grundlage für eine zutreffende Diagnose, da unklar ist, welcher der Allele A und B vom Betroffenen stammt. In diesem Fall wird bei der vorliegenden Ausführungsform das Blut des nicht betroffenen Elternteils des Kindes untersucht. Dabei wird herausgefunden, welche Allele vom nicht betroffenen Elternteil stammen. Hat im vorliegenden Fall der nicht betroffene Elternteil die Allele A und C, so ist klar, dass das Allel A nur von dem nicht betroffenen Elternteil stammen kann. Ebenso wäre in diesem Beispiel dann klar, dass das Allel B, welches wahrscheinlich ausschließlich für die Erkrankung des betroffenen Elternteils verantwortlich ist, an das Kind weitervererbt wurde. Das Kind hätte in diesem Falle daher ein erhöhtes Risiko zu erkranken.

Bei NF2 hat es sich als vorteilhaft erwiesen, mindestens einen der Marker CRYB2, D22S275, NF2CA3, D22S268, D22S430 zu verwenden.

Die Gesamtheit des durch das erfindungsgemäße Verfahren zur Verfügung gestellten und graphisch aufgearbeiteten Daten ermöglichen es dann dem die Diagnose schließlich treffenden Arzt zu beurteilen, ob bei dem untersuchten Risikopatienten die Krankheit ausgeschlossen werden kann. Denn stellt der untersuchende Arzt beispielsweise fest, daß das Allel von dem Verwandten nicht geerbt wurde, welches in dem Tumor noch vorhanden ist (wie beispielsweise in dem unten dargestellten Beispiel), so kann das Auftreten der entsprechenden Tumorsuppressorgenerkrankung ausgeschlossen werden.

Darüber hinaus kann jedoch auch dann, wenn der Risikopatient das im Tumor verbliebene Allel geerbt hat, von dem Arzt eine Diagnose getroffen werden.

Es ergeben sich in einem solchen Fall zwei Diagnosemöglichkeiten:
1. Wenn in einem solchen Fall beispielsweise die Großeltern des Risikopatienten bereits von der entsprechenden Tumorsuppressorgenerkrankung betroffen waren, kann angenommen werden, daß der Risikopatient die Krankheit ebenfalls ererbt hat.
2. Wenn jedoch die Krankheit bei den betroffenen Eltern erstmalig (sporadisch) aufgetreten ist, besteht andererseits die Möglichkeit, daß es sich um eine Mosaikbildung mit einer Wahrscheinlichkeit von 20 bis 30% handelt, so daß die genetische Veränderung bei den an der Tumorsuppressorgenerkrankung erkrankten Eltern mit verminderter Wahrscheinlichkeit an den Risikopatienten vererbt wurde.

Das erfindungsgemäße Verfahren läßt sich insbesondere zur Vorbereitung der präsymptomatischen und pränatalen Diagnose von Neurofibromatosen anwenden. Im folgenden wird als Ausführungsbeispiel der vorliegenden Erfindung die Anwendung des Verfahrens auf Neurofibromatose-Risiko-Patienten erläutert:

### 1. Material: Blut und Tumormaterial

Von einer Vielzahl von Patienten liegt Tumormaterial in Form von Paraffinblöcken aufgrund der Operation der entstellenden Hauttumoren bei NF1 vor. Aus kosmetischen Gründen kann eine Tumorentfernung jederzeit komplikationslos erfolgen. Darüber hinaus gibt es einen Bestand an eingefrorenem Tumormaterial. Alle von einem Patienten vorhandenen Tumoren werden in die erfindungsgemäße Analyse einbezogen.

### 2. DNA-Isolierung:

Aus dem Blut und dem Tumormaterial des Betroffenen wird DNA mit dem QlAquick Blood und dem QlAquick Tissue Kit der Firma Qiagen isoliert. Das Verfahren ist in der Anleitung für die Kits von der Firma beschrieben.

### 3. Polymorphe Marker

Vier polymorphe Mikrosatelliten-Marker, die in den Introns 27 und 38 des NF1 -Gens liegen, werden aus der Blut- und Tumor-DNA amplifiziert. Bei jedem Patienten sollten mindestens zwei dieser vier Marker informativ sein; ansonsten wird ein zusätzlicher Marker verwendet.

### 4. Vermehrung der Marker

Mit Primern (Oligonukleotide) von ca. 20-24 Basenpaaren, die das Ende der in ihrer Länge variablen DNA-Abschnitte flankieren, werden die Marker mittels PCR (Polymerase Chain Reaction) vermehrt. Dieser Vermehrungsprozeß wird in 10 *µ*l Reaktionslösung durchgeführt und enthält Blut- oder Tumor-DNA, Oligonukleotide als Primer, dNTPs, Puffer, Taq-Polymerase und Wasser. Die PCR wird in einem Thermocycler durchgeführt. Für die folgende Analyse werden die Primer mit Fluoreszens-Farbstoff markiert.

### 5. Analyse der vermehrten Marker

0,1-1 *µ*l von jedem der vier vermehrten Marker wird zusammengemischt. Dazu wird 0,5 ROX-Längen-Standard (Fa. ABI) und 12 *µ*l demineralisiertes Formamid zugegeben. Diese Probe wird Hitze-Denaturierung auf die Kapillare eines Genetic Analysers AB1310 aufgeladen. Bei diesem Trennungsverfahren werden die DNA-Abschnitte nach ihren unterschiedlichen Längen aufgetrennt. Die Ergebnisse werden mit dem GeneScan Programm (Fa. ABI) graphisch dargestellt. Die DNA-Abschnitte kommen damit in ihrer unterschiedlichen Länge und Menge zur Darstellung.

### 6. Auswertung der Marker-Analyse

Wenn ein Marker bei einer Person informativ ist, ergeben sich zwei Spitzen bei der graphischen Darstellung der Marker aus dem Blut der Person. Diese Daten werden mit den Ergebnissen aus der Analyse des Tumormaterials verglichen. Wenn ein Tumor nur eine Spitze (Peak) bezüglich dieses Markers oder ein Ungleichgewicht der beiden Spitzen der Marker zeigt, bedeutet dies, daß der Tumor ein Allel verloren hat.

7. Zur Vorbereitung der Diagnose läßt sich dieses Ergebnis dann mit einer entsprechend vorbereiteten Auftragung der Marker aus dem Blut eines Risikopatienten vergleichen. Weiterhin werden die Marker aus dem Blut des gesunden Elternteils entsprechend vorbereitet.

Zur Verdeutlichung der Vorteile der Erfindung wird ein beispielhaftes Ergebnis des erfindungsgemäßen Verfahrens anhand der beiliegenden Zeichnung erläutert. Die Zeichnung zeigt:
- Fig. 1: eine Darstellung von nach ihrer Länge aufgetrennten DNA-Mikrosatelliten-Markern, die aus der DNA aus dem Blut eines Betroffenen amplifiziert wurden;
- Fig. 2: eine Darstellung der Längenauftragung derselben Marker wie aus Fig. 1, die aus Tumormaterial des Betroffenen amplifiziert wurden; und
- Fig. 3: eine Darstellung der Auftragung der Länge nach von denselben Markern, die aus der DNA aus dem Blut eines Nachkommens des Betroffenen gemäß den Fig. 1 und 2 amplifiziert wurden.

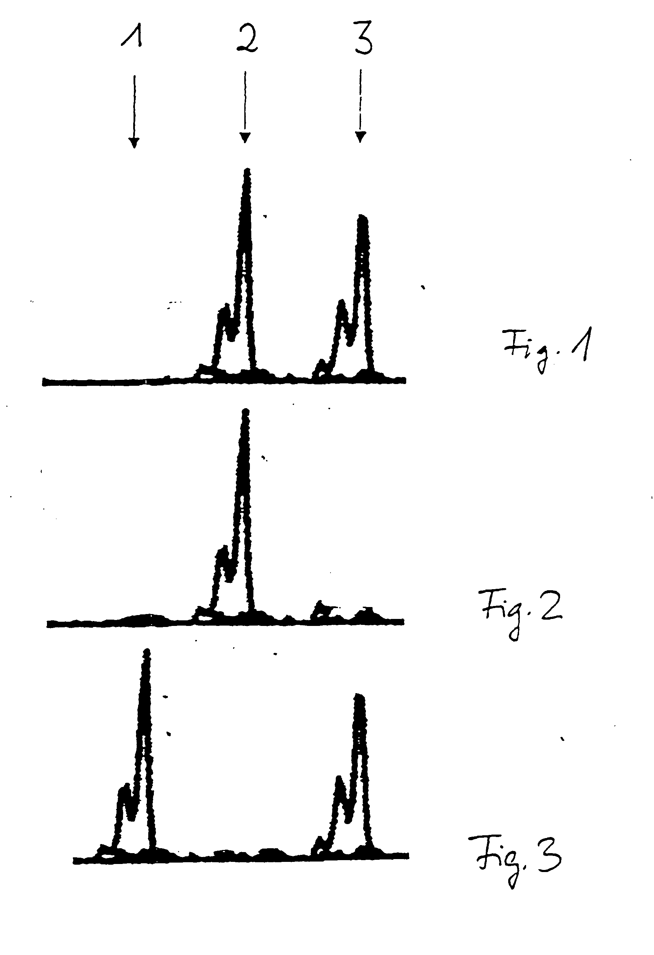

Die Fig. 1 zeigt eine Darstellung von nach ihrer Länge aufgetrennten DNA-Mikrosatelliten-Markern, die aus der DNA aus dem Blut eines Betroffenen amplifiziert wurden. Die graphische Darstellung repräsentiert jeweils das Vorhandensein der Allele, die in der Fig. mit Allel 2 und Allel 3 bezeichnet sind. Die Fig. 2 zeigt eine Darstellung der Längenauftragung derselben Marker wie aus Fig. 1, die aus Tumormaterial des Betroffenen amplifiziert wurden. Man erkennt, daß das Allel, welches in der Fig. 1 mit Allel 3 bezeichnet ist, in dem Tumor des Betroffenen verloren gegangen ist. Die Fig. 3 zeigt eine Darstellung der Auftragung der Länge nach von denselben Markern, die aus der DNA aus dem Blut eines Nachkommens des Betroffenen gemäß den Fig. 1 und 2 amplifiziert wurden. Aus der Fig. 3 ist zu erkennen, daß der Nachkomme des Betroffenen das in dem Tumor noch vorhandene Allel 2 nicht geerbt hat. Der Nachkomme hat von den Allelen 2 und 3 nur das in dem Tumor verloren gegangene Allel 3 geerbt. Als weiteres Allel hat der Nachkomme noch das Allel 1 von dem anderen Elternteil geerbt. Zur Vorbereitung der Diagnose des Nachkommen läßt sich somit festhalten, daß das wahrscheinlich ausschließlich für die Erkrankung verantwortliche Allel nicht an den Nachkommen vererbt wurde.

## Patentansprüche

1. Verfahren zur Ermittlung von Daten zur Vorbereitung der präsymptomatischen oder pränatalen Diagnose von Neurofibromatosen vom typ 1 eines Risikopatienten, mit den Schritten:
a) aus Tumormaterial eines an der Tumorsuppressorgenerkrankung erkrankten Verwandten des Risikopatienten wird Tumor-DNA isoliert,
b) aus Blut des Verwandten wird Blut-DNA isoliert,
c) mindestens zwei polymorphe DNA-Mikrosatelliten-Marker werden aus dem Tumormaterial amplifiziert,
d) die polymorphen DNA-Mikrosatelliten-Marker werden aus dem Blut amplifiziert,
e) die mindestens zwei polymorphen DNA-Mikrosatelliten-Marker aus dem Tumormaterial werden nach Länge aufgetrennt,
f) die mindestens zwei polymorphen DNA-Mikrosatelliten-Marker aus dem Blut werden nach Länge aufgetrennt,
g) die Längen der polymorphen DNA-Mikrosatelliten-Marker aus dem Tumormaterial werden dargestellt,
h) die Längen der polymorphen DNA-Mikrosatelliten-Marker aus dem Blut werden dargestellt,
i) die Schritte b), d), f), h) werden mit Blut des Risikopatienten wiederholt,
wobei es sich bei den Markern um ein Neurofibromatosegen flankierende oder um intragenische Marker handelt,
wobei es sich bei dem Verwandten um ein Elternteil des Risikopatienten handelt.

2. Verfahren nach Anspruch 1,
wobei es sich bei den mindestens zwei polymorphen Markern um kurze, bevorzugt bis etwa 300 bp lange, Marker handelt.

3. Verfahren nach einem der vorstehenden Ansprüche,
wobei mindestens drei, weiter bevorzugt vier, verschiedene Marker verwendet werden.

4. Verfahren nach einem der vorstehenden Ansprüche,
wobei mindestens einer der Marker in dem Intron 27 des Neurofibromatose Typ 1-Gens liegt.

5. Verfahren nach einem der vorstehenden Ansprüche,
wobei mindestens einer der Marker in dem Intron 38 des Neurofibromatose Typ 1-Gens liegt.

6. Verfahren nach einem der vorstehenden Ansprüche,
mit den weiteren Schritten:
Die Schritte a), c), e), g) werden mindestens einmal wiederholt.

7. Verfahren nach Anspruch 6,
mit den weiteren Schritten:
Die Schritte a), c), e), g) werden mit mindestens einem weiteren Tumor des Verwandten wiederholt.

8. Verfahren nach einem der vorstehenden Ansprüche,
mit den weiteren Schritten:
Die Schritte b), d), f), h) werden auch mit dem Blut des nicht betroffenen Elternteils durchgeführt, wenn es sich bei dem Risikopatienten um ein Kind beider Elternteile handelt.

## Claims

1. Method for determining data for the preparation of the presymptomatic or prenatal diagnosis of neurofibromatosis of type 1 in an at-risk patient, comprising the steps of:
a) isolating tumour DNA from tumour material of a relative of the at-risk patient, which relative suffers from the tumour suppression gene disorder,
b) isolating blood DNA from blood of the relative,
c) amplifying at least two polymorphic DNA microsatellite markers from the tumour material,
d) amplifying the polymorphic DNA microsatellite markers from the blood,
e) separating the at least two polymorphic DNA microsatellite markers from the tumour material according to length,
f) separating the at least two polymorphic DNA microsatellite markers from the blood according to length,
g) displaying the lengths of the polymorphic DNA microsatellite markers from the tumour material,
h) displaying the lengths of the polymorphic DNA microsatellite markers from the blood,
i) repeating steps b), d), f), h) with blood of the at-risk patient,
wherein the markers are markers flanking a neurofibromatosis gene or are intragenic markers,
wherein the relative is a parent of the at-risk patient.

2. Method according to claim 1,
wherein the at least two polymorphic markers are short markers, preferably markers up to approximately 300 bp in length.

3. Method according to one of the preceding claims,
wherein at least three, more preferably four, different markers are used.

4. Method according to any one of the preceding claims,
wherein at least one of the markers is situated in intron 27 of the neurofibromatosis type 1 gene.

5. Method according to any one of the preceding claims,
wherein at least one of the markers is situated in intron 38 of the neurofibromatosis type 1 gene.

6. Method according to any one of the preceding claims,
comprising the further steps of:
repeating steps a), c), e), g) at least once.

7. Method according to claim 6,
comprising the further steps of:
repeating steps a), c), e), g) with at least one further tumour of the relative.

8. Method according to any one of the preceding claims,
comprising the further steps of:
performing steps b), d), f), h) also with the blood of the non-affected parent if the at-risk patient is a child of both parents.

## Revendications

1. Procédé d'obtention de données servant à préparer le diagnostic pré-symptomatique ou pré-natal de la neurofibromatose de type 1 d'un patient à risque avec les étapes consistant en ce que :
a) de l'ADN tumoral est isolé à partir de la matière tumorale d'un parent du patient à risque souffrant d'une maladie des gènes suppresseurs de tumeur,
b) de l'ADN sanguin est isolé à partir du sang du parent,
c) au moins deux marqueurs d'ADN microsatellites polymorphes sont amplifiés à partir de la matière tumorale,
d) les marqueurs d'ADN microsatellites polymorphes sont amplifiés à partir du sang,
e) les au moins deux marqueurs d'ADN microsatellites polymorphes sont séparés de la matière tumorale selon leur taille,
f) les au moins deux marqueurs d'ADN microsatellites polymorphes sont séparés du sang selon leur taille,
g) les tailles des marqueurs d'ADN microsatellites polymorphes sont représentées à partir de la matière tumorale,
h) les tailles des marqueurs d'ADN microsatellites polymorphes sont représentées à partir du sang,
i) les étapes b), d), f), h) sont répétées avec du sang du patient à risque, sachant qu'il s'agit pour les marqueurs d'un marqueur intragénique ou flanqué d'un gène de la neurofibromatose,
et qu'il s'agit dans le cas des parents d'un des parents du patient à risque.

2. Procédé selon la revendication 1,
sachant qu'il s'agit de préférence dans le cas d'au moins deux marqueurs polymorphes de marqueurs courts, de préférence d'une longueur d'environ 300 bp.

3. Procédé selon l'une quelconque des revendications précédentes,
sachant qu'au moins trois, de manière encore préférée quatre marqueurs différents sont utilisés.

4. Procédé selon l'une quelconque des revendications précédentes,
sachant qu'au moins un des marqueurs se situe dans l'intron 27 du gène de la neurofibromatose de type 1.

5. Procédé selon l'une quelconque des revendications précédentes,
sachant qu'au moins un des marqueurs se situe dans l'intron 38 du gène de la neurofibromatose de type 1.

6. Procédé selon l'une quelconque des revendications précédentes,
avec les autres étapes consistant en ce que :
les étapes a), c), e), g) sont répétées au moins une fois.

7. Procédé selon la revendication 6,
avec les autres étapes consistant en ce que :
les étapes a), c), e), g) sont répétées avec au moins une autre tumeur du parent.

8. Procédé selon l'une quelconque des revendications précédentes,
avec les autres étapes consistante en ce que :
les étapes b), d), f), h) sont également exécutées avec le sang de l'un des parents non concerné, lorsqu'il s'agit en ce qui concerne le patient à risque d'un enfant des deux parents.
